(19) Europäisches Patentamt
European Patent Office
Office européen des brevets

(11) EP 4 538 265 A1

(12) # EUROPEAN PATENT APPLICATION
published in accordance with Art. 153(4) EPC

(43) Date of publication:
**16.04.2025 Bulletin 2025/16**

(21) Application number: **23819219.9**

(22) Date of filing: **08.06.2023**

(51) International Patent Classification (IPC):
***C07D 401/14*** $^{(2006.01)}$ ***A61P 35/00*** $^{(2006.01)}$
***A61K 31/506*** $^{(2006.01)}$

(52) Cooperative Patent Classification (CPC):
**A61K 31/506; A61P 35/00; C07D 401/14**

(86) International application number:
**PCT/CN2023/099164**

(87) International publication number:
**WO 2023/237055 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.06.2022 CN 202210650673**

(71) Applicant: **Chia Tai Tianqing Pharmaceutical Group Co., Ltd.**
**Lianyungang, Jiangsu 222062 (CN)**

(72) Inventors:
- **YU, Ding**
**Lianyungang, Jiangsu 222062 (CN)**
- **WANG, Xunqiang**
**Lianyungang, Jiangsu 222062 (CN)**
- **CUI, Sisi**
**Lianyungang, Jiangsu 222062 (CN)**

(74) Representative: **Grünecker Patent- und Rechtsanwälte**
**PartG mbB**
**Leopoldstraße 4**
**80802 München (DE)**

(54) **USE OF CDK4/6 INHIBITOR IN TREATMENT OF DEDIFFERENTIATED LIPOSARCOMA**

(57) The present disclosure belongs to the technical field of medicines, and relates to the use of a CDK4/6 inhibitor in treatment of dedifferentiated liposarcoma, in particular to the use of a compound of formula (I) in treatment of dedifferentiated liposarcoma.

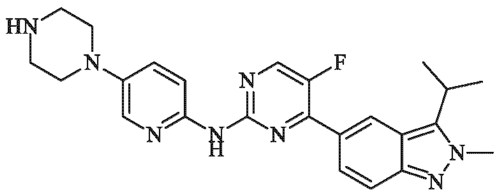

(I)

EP 4 538 265 A1

## Description

### CROSS-REFERENCE TO RELATED APPLICATIONS

The present application claims priority and benefit to the Chinese Patent Application No. 202210650673.6 filed with China National Intellectual Property Administration on Jun. 09, 2022, the disclosure of which is incorporated herein by reference in its entirety.

### TECHNICAL FIELD

The present disclosure belongs to the technical field of medicines and relates to use of a CDK4/6 inhibitor for treating dedifferentiated liposarcoma, in particular to use of a compound of formula (I) for treating dedifferentiated liposarcoma.

### BACKGROUND

Cyclin-dependent kinase (CDK) 4/6 is a key regulator of the cell cycle and is capable of triggering the progression of the cell cycle from the growth phase (G1 phase) to the DNA replication phase (S1 phase). In the process of cell proliferation, the complex formed by CDK4/6 and cyclin D can phosphorylate the retinoblastoma protein (Rb). Once the tumor suppressor protein Rb is phosphorylated, it can release transcription factor E2F to which it tightly binds in the unphosphorylated state. E2F activates further transcription to promote the cell cycle to pass the restriction point (R point) and progress from the G1 phase to the S phase. Therefore, the inhibition of CDK4/6 makes it unable to form a cyclin D-CDK4/6 complex, which can block the progression of the cell cycle from the G1 phase to the S phase, thereby achieving the purpose of inhibiting tumor proliferation. A substituted 2-hydrogen-pyrazole derivative as a selective CDK4/6 inhibitor is disclosed in WO2016141881, and a compound of formula (I) with the following structure is also specifically disclosed:

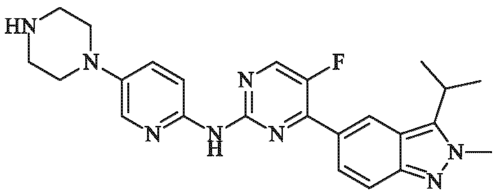

(I)

The age of onset for dedifferentiated liposarcoma (DDLS) is mainly between 60 and 80 years, and the gender distribution is relatively balanced. However, the analysis of 3573 cases performed by the National Cancer Database (NCDB) revealed that 65% of cases were male. It was found that 85% of DDLS were new primary tumors, and the remaining 15% of cases were secondary to well-differentiated liposarcoma (WDLS), with an average 7.7-year recurrence interval. The most primary site of DDLS is retroperitoneum, and less frequent sites include extremities, areas around the testis, head and neck, and chest, and DDLS is rarely found in soft tissues. DDLS is less invasive than other polymorphic sarcomas, the local recurrence rate is about 41%-52%, the metastasis rate is 15%-30%, and the 5-year disease-related mortality rate is 28%-30%.

At present, radical surgical resection is still the main means for DDLS treatment, and in the aspect of improving the local control rate, the 3-year local recurrence rate without radiotherapy is 49%, while the local recurrence rate with radiotherapy is 34%, but the overall survival rate is not influenced. For patients with DDLS characterized by large limb/trunk wall (> 5 cm) and deep infiltration, the use of the systemic therapy (neoadjuvant therapy) should be considered. However, because DDLS tends to respond poorly to first-line chemotherapeutic drugs, the options available for recurrent patients are relatively limited. Therefore, it is necessary to develop a drug for DDLS.

### SUMMARY

In one aspect, the present disclosure provides a compound of formula (I) or a pharmaceutically acceptable salt thereof for use in treating or preventing dedifferentiated liposarcoma,

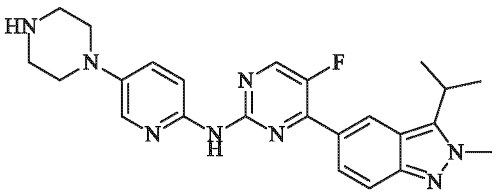

(I)

.

In another aspect, the present disclosure provides a pharmaceutical composition for use in treating or preventing dedifferentiated liposarcoma, which comprises a compound of formula (I) or a pharmaceutically acceptable salt thereof.

In some embodiments of the present disclosure, the pharmaceutical composition is packaged in a kit, and the kit further comprises an instruction for using the compound of formula (I) or the pharmaceutically acceptable salt thereof to treat or prevent dedifferentiated liposarcoma.

In some embodiments of the present disclosure, in the pharmaceutical composition, the pharmaceutically acceptable salt of the compound of formula (I) is maleate, such as monomaleate of the compound of formula (I). In some embodiments of the present disclosure, the pharmaceutical composition comprises 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I).

In some embodiments of the present disclosure, the pharmaceutical composition comprises 150-180 mg of the compound of formula (I) or the pharmaceutically acceptable salt thereof based on the compound of formula (I).

In some embodiments of the present disclosure, the pharmaceutical composition comprises 20 mg, 40 mg, 60 mg, 80 mg, 100 mg, 120 mg, 150 mg, or 180 mg of the compound of formula (I) or the pharmaceutically acceptable salt thereof based on the compound of formula (I).

In some embodiments of the present disclosure, the pharmaceutical composition comprises 60 mg, 120 mg, 150 mg, or 180 mg of the compound of formula (I) or the pharmaceutically acceptable salt thereof based on the compound of formula (I).

In some embodiments of the present disclosure, the pharmaceutical composition comprises 60 mg, 150 mg, or 180 mg of the compound of formula (I) or the pharmaceutically acceptable salt thereof based on the compound of formula (I).

In some embodiments of the present disclosure, the pharmaceutical composition comprises 150 mg or 180 mg of the compound of formula (I) or the pharmaceutically acceptable salt thereof based on the compound of formula (I).

In some embodiments of the present disclosure, the pharmaceutical composition comprises 180 mg of the compound of formula (I) or the pharmaceutically acceptable salt thereof based on the compound of formula (I).

In some embodiments of the present disclosure, in the pharmaceutical composition, the compound of formula (I) or the pharmaceutically acceptable salt thereof is in a single-dose form or in a multi-dose form. In some embodiments of the present disclosure, in the pharmaceutical composition, the compound of formula (I) or the pharmaceutically acceptable salt thereof is in a multi-dose form.

In some embodiments of the present disclosure, in the pharmaceutical composition, the compound of formula (I) or the pharmaceutically acceptable salt thereof is in a daily dose.

In some embodiments of the present disclosure, in the pharmaceutical composition, the compound of formula (I) or the pharmaceutically acceptable salt thereof is in a once-daily dose.

In some embodiments of the present disclosure, in the pharmaceutical composition, the compound of formula (I) or the pharmaceutically acceptable salt thereof is in a once-daily dose, and a dose for each administration is a single dose or multiple doses, typically multiple doses.

In some embodiments of the present disclosure, the pharmaceutical composition comprises the compound of formula (I) or the pharmaceutically acceptable salt thereof in a single dose of 50 mg or 60 mg based on the compound of formula (I). Alternatively, the pharmaceutical composition is in the form of a single-administration formulation, and the pharmaceutical composition comprises 50 mg or 60 mg of the compound of formula (I) or the pharmaceutically acceptable salt thereof based on the compound of formula (I).

In some embodiments of the present disclosure, the pharmaceutical composition comprises the compound of formula (I) or the pharmaceutically acceptable salt thereof in a single dose of 60 mg based on the compound of formula (I). Alternatively, the pharmaceutical composition is in the form of a single-administration formulation, and the pharmaceutical composition comprises 60 mg of the compound of formula (I) or the pharmaceutically acceptable salt thereof based on the compound of formula (I).

In some embodiments of the present disclosure, every 28 days are counted as one treatment cycle.

In some embodiments of the present disclosure, the pharmaceutical composition is a formulation suitable for administration within a single treatment cycle (e.g., a 28-day treatment cycle), and the formulation comprises a

pharmaceutical composition comprising the compound of formula (I) or the pharmaceutically acceptable salt thereof in a total dose of 1680-5040 mg (e.g., 1680 mg, 3360 mg, 4200 mg, 5040 mg, or a range formed by any two of the values) based on the compound of formula (I).

In other embodiments of the present disclosure, the pharmaceutical composition is a formulation suitable for administration within a single treatment cycle (e.g., a 28-day treatment cycle), and the formulation comprises a pharmaceutical composition comprising the compound of formula (I) or the pharmaceutically acceptable salt thereof in a total dose of 4200 mg or 5040 mg based on the compound of formula (I).

In some embodiments of the present disclosure, the pharmaceutical composition is a formulation suitable for administration within a single treatment cycle (e.g., a 28-day treatment cycle), and the formulation comprises a pharmaceutical composition comprising the compound of formula (I) or the pharmaceutically acceptable salt thereof in a total dose of 5040 mg based on the compound of formula (I).

In another aspect, the present disclosure also provides a kit comprising the compound of formula (I) or the pharmaceutically acceptable salt thereof described herein.

In another aspect, the present disclosure also provides a kit of a pharmaceutical composition for use in treating or preventing dedifferentiated liposarcoma, which comprises the compound of formula (I) or the pharmaceutically acceptable salt thereof described herein.

In some embodiments of the present disclosure, in the pharmaceutical composition, the compound of formula (I) or the pharmaceutically acceptable salt thereof is prepared to comprise 50 mg or 60 mg of the compound of formula (I) or the pharmaceutically acceptable salt thereof based on the compound of formula (I) in a unit formulation.

In some embodiments of the present disclosure, in the pharmaceutical composition, the compound of formula (I) or the pharmaceutically acceptable salt thereof is prepared to comprise 60 mg of the compound of formula (I) or the pharmaceutically acceptable salt thereof based on the compound of formula (I) in a unit formulation.

In another aspect, the present disclosure also provides a method for treating or preventing dedifferentiated liposarcoma, which comprises administering to an individual in need thereof a therapeutically effective amount of the compound of formula (I) or the pharmaceutically acceptable salt thereof, for example, administering to an individual in need thereof a therapeutically effective amount of the pharmaceutical composition described above in the present disclosure.

In another aspect, the present disclosure also provides use of the compound of formula (I) or the pharmaceutically acceptable salt thereof for preparing a medicament for treating or preventing dedifferentiated liposarcoma, for example, use of the pharmaceutical composition of the present disclosure for preparing a medicament for treating or preventing dedifferentiated liposarcoma.

In another aspect the present disclosure also provides use of the compound of formula (I) or the pharmaceutically acceptable salt thereof for treating or preventing dedifferentiated liposarcoma, for example, use of the pharmaceutical composition of the present disclosure for treating or preventing dedifferentiated liposarcoma.

In some embodiments of the present disclosure, in the method or the use, a content of the compound of formula (I) or the pharmaceutically acceptable salt thereof in the pharmaceutical composition is a daily dose, and the compound of formula (I) or the pharmaceutically acceptable salt thereof is administered as follows: administering the compound of formula (I) or the pharmaceutically acceptable salt thereof once daily.

In some embodiments of the present disclosure, in the method or the use, a content of the compound of formula (I) or the pharmaceutically acceptable salt thereof in the pharmaceutical composition is a daily dose, wherein the compound of formula (I) or the pharmaceutically acceptable salt thereof is administered in a single-dose form or in a multi-dose form, typically in a multi-dose form; further, the compound of formula (I) or the pharmaceutically acceptable salt thereof is administered once daily in a multi-dose form.

In some embodiments of the present disclosure, in the method or the use, the compound of formula (I) or the pharmaceutically acceptable salt thereof is administered in a daily dose of 60 mg, or in a daily dose of 120 mg, or in a daily dose of 150 mg, or in a daily dose of 180 mg.

In some embodiments of the present disclosure, in the method or the use, the compound of formula (I) or the pharmaceutically acceptable salt thereof is administered in a daily dose of 150 mg or in a daily dose of 180 mg.

In some embodiments of the present disclosure, in the method or the use, the compound of formula (I) or the pharmaceutically acceptable salt thereof is administered in a daily dose of 180 mg.

In some embodiments of the present disclosure, in the method or the use, the compound of formula (I) or the pharmaceutically acceptable salt thereof in the pharmaceutical composition is administered in a multi-dose form consisting of single doses of 50 mg of the compound of formula (I) or the pharmaceutically acceptable salt thereof. In some embodiments of the present disclosure, in the method or the use, the compound of formula (I) or the pharmaceutically acceptable salt thereof is administered based on a regimen of consecutive daily administration.

In some embodiments of the present disclosure, in the method or the use, the compound of formula (I) or the pharmaceutically acceptable salt thereof in the pharmaceutical composition is administered in a multi-dose form consisting of single doses of 60 mg of the compound of formula (I) or the pharmaceutically acceptable salt thereof. In

some embodiments of the present disclosure, in the method or the use, the compound of formula (I) or the pharmaceutically acceptable salt thereof is administered based on a regimen of consecutive daily administration.

In some embodiments of the present disclosure, in the method or the use, a content of the compound of formula (I) or the pharmaceutically acceptable salt thereof in the pharmaceutical composition is a dose per treatment cycle, and the compound of formula (I) or the pharmaceutically acceptable salt thereof is administered as follows: administering the compound of formula (I) or the pharmaceutically acceptable salt thereof daily, wherein the compound of formula (I) or the pharmaceutically acceptable salt thereof is packaged in a single aliquot or in multiple aliquots (e.g., 2, 4, 7, 14, 28 or more aliquots).

In some embodiments of the present disclosure, in the method or the use, 28 days are counted as one treatment cycle, and the compound of formula (I) or the pharmaceutically acceptable salt thereof is administered daily on days 1-28 in each treatment cycle.

In some specific embodiments of the present disclosure, in the method or the use, 28 days are counted as one treatment cycle, and the compound of formula (I) or the pharmaceutically acceptable salt thereof is administered once daily on days 1-28 in each treatment cycle.

In some embodiments of the present disclosure, in the method or the use, 28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 1680-5040 mg. In some embodiments, a total dose of the pharmaceutical composition comprising the compound of formula (I) or the pharmaceutically acceptable salt thereof is selected from the group consisting of 1680 mg, 3360 mg, 4200 mg, 5040 mg, and a range formed by any two of the values. In some embodiments, a total dose of the pharmaceutical composition comprising the compound of formula (I) or the pharmaceutically acceptable salt thereof is preferably 4200 mg or 5040 mg. In some embodiments, a total dose of the pharmaceutical composition comprising the compound of formula (I) or the pharmaceutically acceptable salt thereof is preferably 5040 mg.

In embodiments of the present disclosure, the treatment cycle is repeated as long as the disease is still under control and the administration regimen is clinically tolerable.

In some embodiments of the present disclosure, the dedifferentiated liposarcoma is selected from the group consisting of dedifferentiated liposarcoma difficult to be resected by surgery and dedifferentiated liposarcoma for which surgery is refused.

In some embodiments of the present disclosure, the dedifferentiated liposarcoma is dedifferentiated liposarcoma difficult to be resected by surgery and dedifferentiated liposarcoma for which surgery is refused, for which pathological and imageological examination results suggest the presence of a dedifferentiated liposarcoma component.

In some embodiments of the present disclosure, the dedifferentiated liposarcoma is newly diagnosed dedifferentiated liposarcoma; and/or dedifferentiated liposarcoma having a postoperative residual lesion; and/or locally recurrent or metastatic dedifferentiated liposarcoma; and/or dedifferentiated liposarcoma for which disease progression was imageologically confirmed within past 6 months.

In some embodiments of the present disclosure, the dedifferentiated liposarcoma is a patient having dedifferentiated liposarcoma difficult to be resected by surgery and dedifferentiated liposarcoma for which surgery is refused, and the pathological and imageological examination results suggest the presence of a dedifferentiated liposarcoma component, and the subject is further required to satisfy any one of the following categories: 1) newly diagnosed dedifferentiated liposarcoma; 2) having postoperative residual lesion; 3) locally recurrent or metastatic dedifferentiated liposarcoma; 4) imageologically confirmed disease progression within past 6 months.

In the present disclosure, the "difficult to be resected by surgery" means that an R0/R1 resection can not be achieved by surgery based on the assessment of the researchers, and specifically includes: the tumor is huge or important organs are involved; and/or the tumor is located at a site of an important vascular pathway; and/or the tumor is characterized by multiple metastases and can not be controlled by surgery; and/or the tumor patient also has severe internal medicine diseases that can pose a fatal surgical risk; and/or postoperative recurrence has occurred several times and thus immediate surgery is not suitable.

In the present disclosure, the "newly diagnosed" patient is a treatment-naive subject, which refers to a subject who has received no systemic drug therapy or has not undergone disease relapse within 6 months after postoperative adjuvant therapy.

In the present disclosure, the "disease progression" patient is a treated subject, which refers to a subject who has received first-line systemic drug therapy or has undergone disease relapse within 6 months after postoperative adjuvant therapy.

In some embodiments of the present disclosure, the dedifferentiated liposarcoma has at least one measurable lesion according to RECIST 1.1 criteria.

In other embodiments of the present disclosure, the dedifferentiated liposarcoma has at least one measurable lesion according to RECIST 1.1 criteria, and should be clearly defined as progressive if the measurable lesion is located within an area of previous radiotherapy.

In some embodiments of the present disclosure, all acute toxic effects of previous treatment or surgery in the dedifferentiated liposarcoma patient prior to first administration have been restored to grade ≤ 1 or baseline (according to Common Terminology Criteria for Adverse Events, Version 5.0), except for hair loss and grade 2 peripheral neuropathy.

In other embodiments of the present disclosure, the dedifferentiated liposarcoma patient has previously received treatment by surgical resection (e.g., treatment failure).

In other embodiments of the present disclosure, the dedifferentiated liposarcoma patient has previously received treatment with epirubicin or a pharmaceutically acceptable salt thereof and/or ifosfamide (e.g., treatment failure). In other embodiments of the present disclosure, the dedifferentiated liposarcoma patient has previously received treatment with extended resection of tumor tissues and/or vascular exploration (e.g., treatment failure).

In other embodiments of the present disclosure, the dedifferentiated liposarcoma patient has previously received treatment with a tyrosine kinase inhibitor (e.g., anlotinib) (e.g., treatment failure).

In other embodiments of the present disclosure, the dedifferentiated liposarcoma patient has previously received treatment with systemic chemotherapy (e.g., eribulin) (e.g., treatment failure).

In other embodiments of the present disclosure, the dedifferentiated liposarcoma patient has previously received treatment with adjuvant chemotherapy using an eribulin for injection regimen (e.g., treatment failure).

In other embodiments of the present disclosure, the dedifferentiated liposarcoma patient has previously received treatment with radiotherapy (e.g., treatment failure).

In other embodiments of the present disclosure, the dedifferentiated liposarcoma patient has previously received treatment with an immune checkpoint inhibitor (e.g., an anti-PD-1 antibody or an anti-PD-L1 antibody) (e.g., treatment failure). In other embodiments of the present disclosure, the immune checkpoint inhibitor is selected from camrelizumab.

In other embodiments of the present disclosure, the dedifferentiated liposarcoma patient has previously received treatment with pirarubicin (e.g., treatment failure).

In other embodiments of the present disclosure, the dedifferentiated liposarcoma patient has previously received treatment with surgical resection, epirubicin hydrochloride + ifosfamide, extended resection of tumor tissues + vascular exploration, and anlotinib (e.g., treatment failure).

In other embodiments of the present disclosure, the dedifferentiated liposarcoma patient has previously received treatment with surgical resection and anlotinib (e.g., treatment failure).

In other embodiments of the present disclosure, the dedifferentiated liposarcoma patient has previously received treatment with surgical resection and adjuvant chemotherapy using an eribulin for injection regimen (e.g., treatment failure).

In other embodiments of the present disclosure, the dedifferentiated liposarcoma patient has previously received treatment with surgical resection, radiotherapy, and pirarubicin (e.g., treatment failure).

In other embodiments of the present disclosure, the dedifferentiated liposarcoma patient has previously received treatment with surgical resection and anlotinib + camrelizumab (e.g., treatment failure).

The active components in the pharmaceutical combination of the present disclosure can be formulated independently, or some or all of the active components are co-formulated with a pharmaceutically acceptable carrier and/or excipient. The pharmaceutical combination of the present disclosure may further comprise other therapeutic agents. In some embodiments of the present disclosure, the other therapeutic agents may be therapeutic agents known in the art for cancer, preferably therapeutic agents for dedifferentiated liposarcoma.

In some embodiments of the present disclosure, the cycle is 28 days.

The amount of the compound of formula (I) or the pharmaceutically acceptable salt thereof administered can be determined according to the severity of the disease, the response of the disease, any treatment-related toxicity, and the age and health of a patient.

The compound of formula (I) or the pharmaceutically acceptable salt thereof can be administered via multiple routes of administration including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, intraperitoneal, and intrathecal administrations. In a specific embodiment, the compound of formula (I) or the pharmaceutically acceptable salt thereof is administered orally. In some embodiments of the present disclosure, the compound of formula (I) or the pharmaceutically acceptable salt thereof is administered based on a regimen of consecutive daily oral administration.

In some embodiments of the present disclosure, the compound of formula (I) or the pharmaceutically acceptable salt thereof is administered in the form of a multi-dose oral solid formulation once daily.

Compound of formula (I) or pharmaceutically acceptable salt thereof

The compound of formula (I) of the present disclosure may be administered in its free base form, or in the form of its pharmaceutically acceptable salt, hydrate or prodrug that converts *in vivo* into the form of the compound of formula (I). For example, within the scope of the present disclosure, the pharmaceutically acceptable salt of the compound of formula

(I) can be generated from various organic and inorganic acids according to methods well known in the art.

With respect to the pharmaceutically acceptable salt of the compound of formula (I) described herein, a molar ratio of the compound of formula (I) to an acid radical involved in the formation of the pharmaceutically acceptable salt may be 1:1.

The pharmaceutically acceptable salt of the compound of formula (I) may be maleate of the compound of formula (I) (such as monomaleate of the compound of formula (I)).

The dose of the compound of formula (I) or the pharmaceutically acceptable salt thereof referred to in the present disclosure is based on the amount of the compound of formula (I), unless otherwise stated.

In some embodiments of the present disclosure, the pharmaceutically acceptable salt of the compound of formula (I) is present in a form of a salt of the compound of formula (I).

The compound of formula (I) or the pharmaceutically acceptable salt thereof used in the present disclosure may be prepared by methods known in the art, for example, by reference to WO2016141881.

<u>Pharmaceutical composition of compound of formula (I) or pharmaceutically acceptable salt thereof</u>

In some embodiments of the present disclosure, a single dose of the pharmaceutical composition of the compound of formula (I) or the pharmaceutically acceptable salt thereof is 50 mg or 60 mg based on the compound of formula (I). Alternatively, the pharmaceutical composition of the compound of formula (I) or the pharmaceutically acceptable salt thereof is prepared to comprise 50 mg or 60 mg of the compound of formula (I) or the pharmaceutically acceptable salt thereof based on the compound of formula (I) in a unit formulation.

In some embodiments of the present disclosure, a single dose of the pharmaceutical composition of the compound of formula (I) or the pharmaceutically acceptable salt thereof is 60 mg based on the compound of formula (I). Alternatively, the pharmaceutical composition of the compound of formula (I) or the pharmaceutically acceptable salt thereof is prepared to comprise 60 mg of the compound of formula (I) or the pharmaceutically acceptable salt thereof based on the compound of formula (I) in a unit formulation.

The dosage regimen can be determined comprehensively depending on the activity and toxicity of the medicament, tolerance of a patient, etc.

In some embodiments of the present disclosure, the pharmaceutical composition comprising the compound of formula (I) or the pharmaceutically acceptable salt thereof further comprises a pharmaceutically acceptable excipient. The pharmaceutically acceptable excipient includes a filler, an absorbent, a wetting agent, a binder, a disintegrant, a lubricant, and the like. In some embodiments of the present disclosure, the pharmaceutical composition includes, but is not limited to, a formulation suitable for oral, parenteral and topical administration. In some embodiments, the pharmaceutical composition is a formulation suitable for oral administration. In some embodiments, the pharmaceutical composition is a solid formulation suitable for oral administration. In some embodiments, the pharmaceutical composition includes, but is not limited to, a tablet and a capsule.

In some embodiments of the present disclosure, the pharmaceutical composition is a solid pharmaceutical combination.

In some embodiments of the present disclosure, the pharmaceutical composition of the compound of formula (I) or the pharmaceutically acceptable salt thereof is a solid pharmaceutical composition comprising the compound of formula (I) or the pharmaceutically acceptable salt thereof.

In some embodiments of the present disclosure, the pharmaceutical composition of the compound of formula (I) or the pharmaceutically acceptable salt thereof is a capsule comprising the compound of formula (I) or the pharmaceutically acceptable salt thereof.

The pharmaceutical composition of the present disclosure can be manufactured using methods well known in the art, such as conventional mixing, dissolving, granulating, dragee-making, levigating, emulsifying, and lyophilizing.

A solid oral composition can be prepared by conventional mixing, filling, or tableting. For example, it can be obtained by the following method: mixing the active compounds with solid excipients, optionally grinding the resulting mixture, adding other suitable excipients if desired, and processing the mixture into granules to get the core parts of tablets or dragees. Suitable excipients include, but are not limited to: binders, diluents, disintegrants, lubricants, glidants, sweeteners, flavoring agents, or the like.

<u>Technical effects</u>

Generally, using the compound of formula (I) of the present disclosure will provide:

(1) good efficacy in reducing the growth of tumors or even eliminating the tumors;
(2) treatment that is well tolerated in a patient with few adverse effects and/or complications;
(3) good disease control rate (DCR) in patients treated;

(4) long survival (e.g., median survival time, progression-free survival, or overall survival) in patients treated;
(5) prolonged survival (e.g., median survival time, progression-free survival, or overall survival) in patients treated as compared with those receiving standard chemotherapy;
(6) long duration of response (DOR).

The "clinical benefits" of the compound of formula (I) of the present disclosure include, but are not limited to: high 12-week progression-free survival rate, prolonged progression-free survival (PFS), prolonged overall survival (OS), improved objective response rate (ORR), improved disease control rate (DCR), reduced number and/or degree of adverse effects, decreased distant metastasis rate, decreased local control rate, and the like for clinical patients.

Definitions and explanations

The word "comprise", and variants thereof such as "comprises" or "comprising", and equivalents thereof shall be understood in an open, non-exclusive sense, i.e., "includes but is not limited to", indicating that in addition to the listed elements, components and procedures, other unspecified elements, components and procedures may also be encompassed.
The term "patient" or "individual/subject" refers to a mammal, e.g., a primate (human, macaque, chimpanzee, etc.), rodent (mouse, rat, rabbit, etc.), feline, canine, etc., preferably a human. In some embodiments of the present disclosure, the patient and the individual are patients who have failed or lack a standard treatment.
The term "pharmaceutically acceptable" refers to a carrier or an excipient that is used in the preparation of a pharmaceutical composition. The carrier or the excipient is generally safe, non-toxic, and desirable biologically and otherwise, and inclusion of the substance is acceptable for pharmaceutical use in humans.
The term "therapeutically effective amount" means an amount of a compound that, when administered to a human for treating a disease, is sufficient to treat the disease.
The term "treat", "treating", or "treatment" refers to administering the compound or the formulation described herein so as to ameliorate, alleviate, or eliminate a disease or one or more symptoms associated with the disease, and includes: (i) inhibiting the disease or disease state, i.e., arresting or delaying its progression; and (ii) alleviating the disease or disease state, i.e., causing regression of the disease or disease state.
The term "prevent", "preventing", or "prevention" means administering the compound or formulation described herein to prevent a disease or one or more symptoms associated with the disease, and includes preventing the occurrence of the disease or disease state in a mammal, particularly when such a mammal is predisposed to the disease state but has not yet been diagnosed with it.
As used herein, an "adverse event" (AE) is any adverse and often unintended or undesirable sign (including abnormal laboratory findings), symptom, or disease associated with the use of medical treatment. For example, an adverse event may be associated with activation of the immune system or expansion of immune system cells (e.g., T cells) in response to treatment. The medical treatment may have one or more related AEs, and each AE may have the same or a different severity level. Reference to a method capable of "altering an adverse event" refers to a treatment regimen that decreases the incidence and/or severity of one or more AEs associated with the use of a different treatment regimen.
The use of alternatives (e.g., "or") shall be understood to refer to any one, two, or any combination of the alternatives. As used herein, the indefinite article "a" or "an" shall be understood to mean "one or more" of any listed or enumerated components.
The term "pharmaceutically acceptable excipient" refers to those excipients that do not have a significant irritating effect on an organic entity and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.
The terms "administer", "administration", and "administering" refer to physically introducing the composition comprising a therapeutic agent to an individual using any one of a variety of methods and delivery systems known to those skilled in the art. In certain embodiments, the administration is oral administration.
The term "daily dose" refers to a dose administered to a patient per day.
The term "single dose" or "unit formulation" refers to the smallest unit of packaging of a pharmaceutical product comprising a certain amount of active ingredients; for example, in a box of seven capsules, each capsule is a single dose or a unit formulation; in a box of seven tablets, each tablet is a single dose or a unit formulation.
The term "multiple doses" or "multi-dose" consists of multiple single doses. As used herein, "combined use" or "use in combination" means that two or more active substances may be administered to an individual simultaneously, concurrently, or sequentially in any order as a single formulation.
The term "pharmaceutical composition" refers to a mixture consisting of one or more of the active ingredients or the pharmaceutical combinations thereof of the present disclosure and a pharmaceutically acceptable excipient. The pharmaceutical composition is intended to facilitate the administration of the compound or the pharmaceutical combination thereof of the present disclosure to an individual.

The terms "day", "daily", and the like, when referred to in an administration regimen, refer to the time within a calendar day that starts at midnight and ends at the next midnight.

The term "recurrent" cancer is one that regenerates at an initial site or a distant site after being responsive to initial treatment (e.g., surgery). A "locally recurrent" cancer is one that occurs, after treatment, at the same location as the previously treated cancer.

The term "metastatic" cancer refers to one that spreads from one part of the body (e.g., the lung) to another part of the body.

Herein, unless the context specifies clearly otherwise, singular terms encompass plural referents, and vice versa. Similarly, unless the context specifies clearly otherwise, the word "or" is intended to include "and", and vice versa.

Unless otherwise stated herein, parameter values representing amounts of ingredients or physicochemical properties or reaction conditions and the like are to be understood as being modified in all cases by the term "about". When the term "about" is used to describe the present disclosure, the term "about" indicates that there is an error value; for example, it means varying within a range of $\pm 5\%$, such as $\pm 1\%$ or 0.1%, of a particular value.

All patents, patent applications and other identified publications are explicitly incorporated herein by reference for the purpose of description and disclosure. These publications are provided solely because they were disclosed prior to the filing date of the present disclosure. All statements as to the dates of these documents or descriptions as to the content of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

Examples

The following specific examples are intended to allow those skilled in the art to clearly understand and implement the present disclosure. These specific examples should not be considered as limitations on the scope of the present disclosure, but merely as exemplary descriptions and representatives of the present disclosure.

**Experimental Example 1. Clinical Trial**

In this study, a randomized, double-blind, placebo-controlled parallel trial design or a single arm trial design was employed. For the parallel control trial, after all patients signed the informed consent, subjects eligible for enrollment by screening were randomized (1:1) into two groups, i.e., a test group given the capsule of the compound of formula I and a control group given the placebo of the capsule of the compound of formula I, with 4 weeks (28 days) being 1 cycle; for the single arm trial, the administration regimen was the same as that for the test group of the parallel control trial. The efficacy and safety of the capsule of the compound of formula I in the treatment of dedifferentiated liposarcoma was evaluated by the clinical trials of the present disclosure.

1.1. Inclusion criteria:

1) Subjects with voluntary participation, signed informed consent, and good compliance;
2) Age: 18-75 years old (calculated based on the date of signing the informed consent); ECOG PS score: 0-1; body mass index (BMI) > 18.5 and body weight > 40 kg; expected survival ≥ 3 months;
3) Being a patient having dedifferentiated liposarcoma difficult to be resected by surgery or dedifferentiated liposarcoma for which surgery is refused, as confirmed by multidisciplinary consultation, with the pathological and imageological examination results suggesting the presence of a dedifferentiated liposarcoma component, and the subject being further required to satisfy any one of the following categories:

    1) Newly diagnosed dedifferentiated liposarcoma;
    2) Having postoperative residual lesion;
    3) Locally recurrent or metastatic dedifferentiated liposarcoma;
    4) Imageologically confirmed disease progression within past 6 months;

4) Those having at least one measurable lesion according to RECIST 1.1 criteria, which should be clearly defined as progressive if the measurable lesion is located within an area of previous radiotherapy;
5) All acute toxic effects of previous treatment or surgery prior to first administration of the subject have been restored

to grade ≤ 1 or baseline (according to Common Terminology Criteria for Adverse Events, Version 5.0 [CTCAE v 5.0]), except for hair loss and grade 2 peripheral neuropathy;
6) Main organs function well and the following criteria should be met:

1) Blood routine examination criteria (no blood transfusion and no correction using hematopoietic stimulating drugs within the last 7 days before examination):

a.

$$\text{Hemoglobin (HGB)} \geq 90.0 \text{ g/L;}$$

b.

$$\text{Absolute neutrophil count (ANC)} \geq 1.5\times10^{9}\text{/L;}$$

c.

$$\text{Platelet count (PLT)} \geq 90 \times 10^{9}\text{/L;}$$

2) Biochemical examination criteria:

a. Total bilirubin (TBIL) ≤ 1.5 × upper limit of normal ULN; for patients with Gilbert syndrome, ≤ 3 × ULN;
b. Alanine aminotransferase (ALT) and aspartate aminotransferase (AST) ≤ 3 × ULN. If liver metastasis is accompanied, ALT and AST ≤ 5 × ULN;
c. Serum creatinine (CR) ≤ 178 μmol/L or creatinine clearance rate (CCR) > 50 mL/min;

3) Urine routine examination criteria: the urine routine indicates that urine protein < ++; if the urine protein ≥ ++, the quantitative determination of the urine protein in 24 hours needs to be confirmed to ≤ 1.0 g;
4) Blood coagulation criteria: prothrombin time (PT), activated partial thromboplastin time (APTT), and international normalized ratio (INR) ≤ 1.5 × ULN (not previously received anticoagulant therapy);
5) Heart color ultrasound evaluation: left ventricular ejection fraction (LVEF) ≥ 50%;
6) 12-lead electrocardiogram evaluation: QTc < 450 ms (male) or QTc < 470 ms (female)
7) Female subjects of childbearing age should agree to take necessary contraceptive measures (such as intrauterine devices, contraceptives, or condoms) during the study and for 6 months after the study; serum pregnancy test results should be negative within 7 days before enrollment, and the subjects must be in the non-lactation period; male subjects should agree to take necessary contraceptive measures during the study and for 6 months after the study.

1.2 Study drug

Capsules of the compound of formula (I): strength: 50 mg or 60 mg, provided by Chia Tai Tianqing Pharmaceutical Group Co., Ltd.

1.3 Administration regimen

Capsules of the compound of formula (I): 180 mg/each administration (based on the compound of formula (I)), oral administration at fasting, once daily, and 28 consecutive days of administration as one treatment cycle. Placebo of the capsule of the compound of formula (I): 0 mg/each administration (based on the compound of formula (I)), oral administration at fasting, once daily, and 28 consecutive days of administration as one treatment cycle.

1.4 Evaluation criteria

Efficacy evaluation criteria: disease state was determined using the RECIST 1.1 criteria.
Safety evaluation criteria: the severity of adverse events was determined using the NCI-CTC AE 5.0 criteria. Adverse event record forms including time of occurrence, severity, relevance to the studied treatment, duration, measures taken, and the final state of the subject should be faithfully filled out during the trial.

1.5 Results

1.5.1. Safety

Gastrointestinal reactions (diarrhea, vomiting, and the like) were mainly grade 1-2, and could be controlled after symptomatic treatment; the total incidence rate of TEAEs of grade 3 or above was 9%, and the total safety is good.

1.5.2. Efficacy

Of the 15 subjects who had achieved the evaluation of C3 (12 weeks) efficacy, 9 patients achieved 12-week PFS, with the 12-week PFS rate being 60%. The progression-free survival (PFS), overall survival (OS), objective response rate (ORR), disease control rate (DCR), and duration of response (DOR) of the patients that could be evaluated were all good. The results show that the compound of formula (I) of the present disclosure exhibits clinical benefits.

Representative cases:

| Subject No. | History of previous treatment | Dose group | Administration cycle | Efficacy evaluation | | | |
|---|---|---|---|---|---|---|---|
| | | | | C1 | C3 | C5 | C7 |
| 1 | 1. Surgical resection;<br>2. Epirubicin hydrochloride + ifosfamide;<br>3. Extended resection of tumor tissues and left femoral vascular exploration; 4. Anlotinib; | Capsules of the compound of formula (I): 180 mg/each administration, once daily; 28 days as one treatment cycle. | C7 | SD | SD | SD | SD |
| 2 | 1. Surgical resection;<br>2. Anlotinib; | Same as No. 1 | C5 | SD | SD | SD | -- |
| 3 | 1. Surgical resection;<br>2. Systemic chemotherapy (adjuvant chemotherapy using an eribulin for injection regimen) | Same as No. 1 | C3 | SD | SD | -- | -- |
| 4 | 1. Surgical resection;<br>2. Radiotherapy;<br>3. Pirarubicin; | Same as No. 1 | C3 | SD | SD | -- | -- |
| 5 | 1. Anlotinib + camrelizumab;<br>2. Surgical resection | Same as No. 1 | C3 | SD | SD | -- | -- |

Those skilled in the art will recognize that the scope of the present disclosure is not limited to the various embodiments and examples described above. Instead, various modifications, substitutions, or recombinations can be made without departing from the spirit and conception of the present disclosure, all of which fall within the protection scope of the present disclosure.

## Claims

1. A compound of formula (I) or a pharmaceutically acceptable salt thereof for use in treating or preventing dedifferentiated liposarcoma,

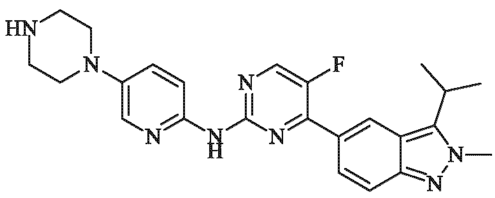

(I)

.

2. A pharmaceutical composition for use in treating or preventing dedifferentiated liposarcoma, comprising a compound of formula (I) or a pharmaceutically acceptable salt thereof,

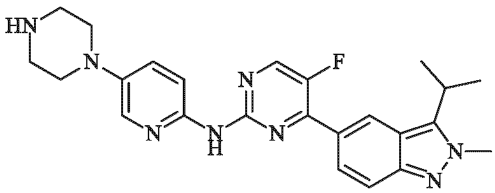

(I)

.

3. The pharmaceutical composition according to claim 2, wherein the pharmaceutical composition is packaged in a kit, and the kit further comprises an instruction for using the compound of formula (I) or the pharmaceutically acceptable salt thereof to treat or prevent dedifferentiated liposarcoma.

4. The pharmaceutical composition according to claim 2, wherein the pharmaceutical composition comprises 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I); or

the pharmaceutical composition comprises 150-180 mg of the compound of formula (I) or the pharmaceutically acceptable salt thereof based on the compound of formula (I); or
the pharmaceutical composition comprises 20 mg, 40 mg, 60 mg, 80 mg, 100 mg, 120 mg, 150 mg, or 180 mg of the compound of formula (I) or the pharmaceutically acceptable salt thereof based on the compound of formula (I); or
the pharmaceutical composition comprises 60 mg, 150 mg, or 180 mg of the compound of formula (I) or the pharmaceutically acceptable salt thereof based on the compound of formula (I); or
the pharmaceutical composition comprises 150 mg or 180 mg of the compound of formula (I) or the pharmaceutically acceptable salt thereof based on the compound of formula (I).

5. The pharmaceutical composition according to claim 2, wherein in the pharmaceutical composition, the compound of formula (I) or the pharmaceutically acceptable salt thereof is in a single-dose form or in a multi-dose form; or
in the pharmaceutical composition, the compound of formula (I) or the pharmaceutically acceptable salt thereof is in a multi-dose form.

6. The pharmaceutical composition according to claim 2, wherein in the pharmaceutical composition, the compound of formula (I) or the pharmaceutically acceptable salt thereof is in a daily dose; or

in the pharmaceutical composition, the compound of formula (I) or the pharmaceutically acceptable salt thereof is in a once-daily dose; or
in the pharmaceutical composition, the compound of formula (I) or the pharmaceutically acceptable salt thereof is in a once-daily dose, and a dose for each administration is a single dose or multiple doses; or
in the pharmaceutical composition, the compound of formula (I) or the pharmaceutically acceptable salt thereof is in a once-daily dose, and a dose for each administration is multiple doses.

7. The pharmaceutical composition according to claim 2, wherein the pharmaceutical composition is a formulation suitable for administration within a single treatment cycle, and the formulation comprises a pharmaceutical composition comprising the compound of formula (I) or the pharmaceutically acceptable salt thereof in a total dose of

1680-5040 mg based on the compound of formula (I); or

the pharmaceutical composition is a formulation suitable for administration within a single treatment cycle, and the formulation comprises a pharmaceutical composition comprising the compound of formula (I) or the pharmaceutically acceptable salt thereof in a total dose of 1680 mg, 3360 mg, 4200 mg, 5040 mg, or a range formed by any two of the values based on the compound of formula (I); or
the pharmaceutical composition is a formulation suitable for administration within a single treatment cycle, and the formulation comprises a pharmaceutical composition comprising the compound of formula (I) or the pharmaceutically acceptable salt thereof in a total dose of 4200 mg or 5040 mg based on the compound of formula (I).

**8.** The pharmaceutical composition according to claim 2, wherein the compound of formula (I) or the pharmaceutically acceptable salt thereof is prepared to comprise 50 mg or 60 mg of the compound of formula (I) or the pharmaceutically acceptable salt thereof based on the compound of formula (I) in a unit formulation.

**9.** A method for treating or preventing dedifferentiated liposarcoma, comprising administering to a subject in need thereof a therapeutically effective amount of the compound of formula (I) or the pharmaceutically acceptable salt thereof according to claim 1 or the pharmaceutical composition according to any one of claims 2-8.

**10.** The method according to claim 9, wherein a content of the compound of formula (I) or the pharmaceutically acceptable salt thereof in the pharmaceutical composition is a daily dose, and the compound of formula (I) or the pharmaceutically acceptable salt thereof is administered as follows: administering the compound of formula (I) or the pharmaceutically acceptable salt thereof once daily; or
a content of the compound of formula (I) or the pharmaceutically acceptable salt thereof in the pharmaceutical composition is a daily dose, and the compound of formula (I) or the pharmaceutically acceptable salt thereof is administered as follows: administering the compound of formula (I) or the pharmaceutically acceptable salt thereof once daily in a multi-dose form.

**11.** The method according to claim 9, wherein the compound of formula (I) or the pharmaceutically acceptable salt thereof is administered in a daily dose of 60 mg, or in a daily dose of 120 mg, or in a daily dose of 150 mg, or in a daily dose of 180 mg.

**12.** The method according to claim 9, wherein 28 days are counted as one treatment cycle, and the compound of formula (I) or the pharmaceutically acceptable salt thereof is administered daily on days 1-28 in each treatment cycle; or

28 days are counted as one treatment cycle, and the compound of formula (I) or the pharmaceutically acceptable salt thereof is administered once daily on days 1-28 in each treatment cycle; or
28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 1680-5040 mg; or
28 days are counted as one treatment cycle, the administration is performed once daily for 28 consecutive days, and a total dose of the pharmaceutical composition comprising the compound of formula (I) or the pharmaceutically acceptable salt thereof is 1680 mg, 3360 mg, 4200 mg, 5040 mg, or a range formed by any two of the values; or
28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 4200 mg or 5040 mg.

**13.** The compound of formula (I) or the pharmaceutically acceptable salt thereof according to claim 1, the pharmaceutical composition according to any one of claims 2-8, or the method according to any one of claims 9-12, wherein the dedifferentiated liposarcoma is selected from the group consisting of dedifferentiated liposarcoma difficult to be resected by surgery and dedifferentiated liposarcoma for which surgery is refused.

**14.** The compound of formula (I) or the pharmaceutically acceptable salt thereof according to claim 1, the pharmaceutical composition according to any one of claims 2-8, or the method according to any one of claims 9-12, wherein the dedifferentiated liposarcoma is newly diagnosed dedifferentiated liposarcoma; and/or dedifferentiated liposarcoma having a postoperative residual lesion; and/or locally recurrent or metastatic dedifferentiated liposarcoma; and/or dedifferentiated liposarcoma for which disease progression was imageologically confirmed within past 6 months.

## INTERNATIONAL SEARCH REPORT

International application No. **PCT/CN2023/099164**

**A. CLASSIFICATION OF SUBJECT MATTER**

C07D401/14(2006.01)i; A61P35/00(2006.01)i; A61K31/506(2006.01)i

According to International Patent Classification (IPC) or to both national classification and IPC

**B. FIELDS SEARCHED**

Minimum documentation searched (classification system followed by classification symbols)

IPC: C07D, A61P, A61K

Documentation searched other than minimum documentation to the extent that such documents are included in the fields searched

Electronic data base consulted during the international search (name of data base and, where practicable, search terms used)

CNABS CNTXT DWPI SIPOABS ENTXT ENTXTC STN: CDK4/6抑制剂, 肿瘤, inhibitor?, cancer, tumor, STN结构式检索, search for structural formula in STN

**C. DOCUMENTS CONSIDERED TO BE RELEVANT**

| Category* | Citation of document, with indication, where appropriate, of the relevant passages | Relevant to claim No. |
|---|---|---|
| X | WO 2016141881 A1 (MEDSHINE DISCOVERY INC.) 15 September 2016 (2016-09-15) description, pages 2-4, embodiment 3 | 1-14 |
| X | WO 2018045993 A1 (CHIA TAI TIANQING PHARMACEUTICAL GROUP CO., LTD. et al.) 15 March 2018 (2018-03-15) description, pages 2 and 5-7 | 1-14 |
| X | WO 2021259203 A1 (CHIA TAI TIANQING PHARMACEUTICAL GROUP CO., LTD.) 30 December 2021 (2021-12-30) description, pages 1-2 | 1-14 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 September 2023** | **14 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/099164**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9-14**
because they relate to subject matter not required to be searched by this Authority, namely:

Claims 9-14 relate to a method for treatment of a disease. The present report is provided according to the technical solution of the preparation use of a corresponding compound or composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/099164**

| Patent document cited in search report | Publication date (day/month/year) | Patent family member(s) | Publication date (day/month/year) |
|---|---|---|---|
| WO 2016141881 A1 | 15 September 2016 | KR 20180005160 A | 15 January 2018 |
| | | HK 1244803 A1 | 17 August 2018 |
| | | EP 3269715 A1 | 17 January 2018 |
| | | EP 3269715 A4 | 08 August 2018 |
| | | EP 3269715 B1 | 13 May 2020 |
| | | BR 112017019286 A2 | 02 May 2018 |
| | | BR 112017019286 B1 | 14 February 2023 |
| | | ES 2806206 T3 | 16 February 2021 |
| | | US 2018072707 A1 | 15 March 2018 |
| | | US 9969719 B2 | 15 May 2018 |
| | | CA 2978363 A1 | 15 September 2016 |
| | | TW 201639831 A | 16 November 2016 |
| | | TWI 688559 B | 21 March 2020 |
| | | AU 2016228660 A1 | 19 October 2017 |
| | | AU 2016228660 B2 | 07 May 2020 |
| | | RU 2017131559 A3 | 11 April 2019 |
| | | RU 2017131559 A | 29 May 2020 |
| | | JP 2018507883 A | 22 March 2018 |
| | | JP 6726677 B2 | 22 July 2020 |
| | | KR 20180005160 A | 15 January 2018 |
| | | HK 1244803 A1 | 17 August 2018 |
| | | EP 3269715 A1 | 17 January 2018 |
| | | EP 3269715 A4 | 08 August 2018 |
| | | EP 3269715 B1 | 13 May 2020 |
| | | BR 112017019286 A2 | 02 May 2018 |
| | | BR 112017019286 B1 | 14 February 2023 |
| | | ES 2806206 T3 | 16 February 2021 |
| | | US 2018072707 A1 | 15 March 2018 |
| | | US 9969719 B2 | 15 May 2018 |
| | | CA 2978363 A1 | 15 September 2016 |
| | | TW 201639831 A | 16 November 2016 |
| | | TWI 688559 B | 21 March 2020 |
| | | AU 2016228660 A1 | 19 October 2017 |
| | | AU 2016228660 B2 | 07 May 2020 |
| | | RU 2017131559 A3 | 11 April 2019 |
| | | RU 2017131559 A | 29 May 2020 |
| | | JP 2018507883 A | 22 March 2018 |
| | | JP 6726677 B2 | 22 July 2020 |
| WO 2018045993 A1 | 15 March 2018 | JP 2019526605 A | 19 September 2019 |
| | | JP 7100625 B2 | 13 July 2022 |
| | | US 2019194168 A1 | 27 June 2019 |
| | | US 10626107 B2 | 21 April 2020 |
| | | EP 3502103 A1 | 26 June 2019 |
| | | EP 3502103 A4 | 19 February 2020 |
| | | EP 3502103 B1 | 06 April 2022 |
| | | JP 2019526605 A | 19 September 2019 |
| | | JP 7100625 B2 | 13 July 2022 |
| | | US 2019194168 A1 | 27 June 2019 |
| | | US 10626107 B2 | 21 April 2020 |
| | | EP 3502103 A1 | 26 June 2019 |

Form PCT/ISA/210 (patent family annex) (July 2022)

**INTERNATIONAL SEARCH REPORT**
**Information on patent family members**

International application No.
**PCT/CN2023/099164**

| Patent document cited in search report | Publication date (day/month/year) | Patent family member(s) | Publication date (day/month/year) |
|---|---|---|---|
| | | EP 3502103 A4 | 19 February 2020 |
| | | EP 3502103 B1 | 06 April 2022 |
| WO 2021259203 A1 | 30 December 2021 | EP 4159728 A1 | 05 April 2023 |
| | | EP 4159728 A1 | 05 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210650673
- WO 2016141881 A